# FASCICULE DE BREVET EUROPEEN

(11) **EP 0 489 640 B1**
(45) Date de publication et mention de la délivrance du brevet: **02.10.1996**
(21) Numéro de dépôt: 91403263.6
(22) Date de dépôt: 03.12.1991
(51) Int. Cl.: A61K 31/215

(54) **Utilisation de phényléthanolaminotétralines pour la préparation de médicaments antidépressifs et anti-stress**
Verwendung von Phenyläthanolaminotetralinen zur Herstellung eines antidepressiven und Antistress-Arzneimittels
Use of phenylethanolaminotetralines for preparing antidepressant and anti-stress medicines

(30) Priorité: 04.12.1990 FR 9015171
(43) Date de publication de la demande: 10.06.1992
(73) Titulaire: SANOFI, F-75008 Paris (FR)
(72) Inventeur: Keane, Peter-Eugène, F-31120 Roquettes (FR); Bianchetti, Alberto, I-20122 Milano (IT); Simiand, Jacques, F-31600 Muret (FR); Croci, Tiziano, I-20154 Milano (IT)
(74) Mandataire: Gillard, Marie-Louise

(56) Documents cités:
- EP-A- 0 303 546
- WO-A-90/07490
- CH-A- 637 373
- FR-A- 2 584 712
- BRITISH JOURNAL OF PHARMACOLOGY, vol. 100, no. 4, août 1990, pages 831-839, Macmillan Press Ltd; A. BIANCHETTI et al.: "In vitro inhibition of intestinal motility by phenylethanolaminotetralines: evidence of atypical beta-adrenoceptors in rat colon"

## Description

La présente invention concerne l'utilisation de certaines phényléthanolaminotétralines dans la préparation de médicaments destinés au traitement ou à la prophylaxie de la dépression et du stress.

Plus particulièrement, un premier objet de la présente invention est l'utilisation d'au moins une phényléthanolaminotéraline de formule générale (I) 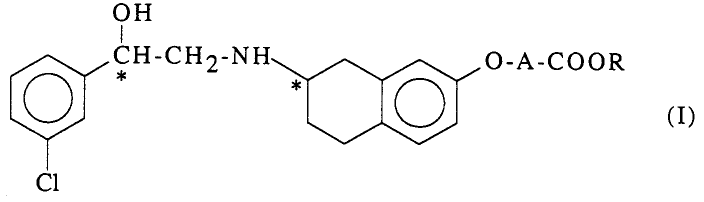
dans laquelle :
A représente un groupe (C₁-C₄)alkylène, et
R répresente un atome d'hydrogène ou un groupe (C₁-C₄) alkyle,
ou un de ses sels pharmaceutiquement acceptables, pour la préparation de médicaments destinés au traitement ou à la prophylaxie de la dépression et du stress.

Les composés de formule (I) sont déjà connus dans l'art antérieur en tant qu'agents lypolytiques et spasmolytiques intestinaux agissant par un mécanisme de stimulation séléctive de récepteurs β atypiques (ni β₁, ni β₂) présents dans l'intestin. Leur préparation et leurs caractéristiques ont été décrites en particulier dans EP-A-211721, EP-A-303545, EP-A-303546 et EP-A-383686.

Il y a une quinzaine d'années, un effet sédatif a été mis clairement en évidence pour les β-stimulants classiques (*A. Ksiazek et al., J. Pharmacol. Pharm., 1974*, *26, 287-295*), et des travaux plus récents ont montré aussi un effet antidépresseur pour certains produits β₂-stimulants, par exemple clenbutérol et salbutamol, effet qui pourrait être lié à l'activité stimulante β-adrénergique de ces produits exercée au niveau central. Malgré ces résultats pharmacologiques prometteurs, les effets secondaires dans le domaine cardiovasculaire rendent l'usage clinique des β-stimulants conventionnels extrêmement difficile.

On vient de trouver à présent que les produits de formule (I), qui exercent une activité stimulante sur les récepteurs β atypiques, n'ont presque aucune activité sur les récepteurs β₁ et aucune, ou dans certains cas une activitée modérée, sur les récepteurs β₂, sont thérapeutiquement très interessants en tant qu'agents antidépresseurs ayant une activité au moins comparable à celle des puissants β₂-agonistes classiques mentionnés ci-dessus et étant dépourvus des effets secondaires inacceptables (notamment tachycardie) observés avec ces derniers.

Les proprietés des composés de formule (I) ont été étudiées à l'aide d'une batterie de tests habituellement utilisés en pharmacologie et généralement considérés en tant que prévisionnels chez l'animal d'une activité antidépressive chez l'homme (*P. Simon*, *Thérapie*, *1973, 28, 209-223*).

Plus particulièrement, les composés de formule (I) ont été essayés chez la souris en comparaison avec le clenbutérol et le salbutamol dans les quatre tests suivants :
- Antagonisme aux effets hypothermisants de l'apomorphine
- Antagonisme aux effets hypothermisants de la réserpine
- Antagonisme aux effets hypothermisants de l'oxotrémorine
- Accroissement de la toxicité à la yohimbine

On a observé aussi les modifications éventuelles du comportement et de l'activité motrice chez des souris après traitement avec des doses croissantes des produits à tester.

Les composés de formule (I) possèdent des effets communs aux produits β-agonistes classiques utilisés comme produits de référence tels que le clenbutérol et le salbutamol: antagonisme aux hypothermies exercées chez la souris par la réserpine, l'oxotrémorine et l'apomorphine à forte dose, et une potentialisation de la toxicité de la yohimbine. Contrairement au clenbutérol et au salbutamol, les composés de formule (I) ne produisent presqu'aucune sédation et aucune diminution de l'activité motrice spontanée chez les souris.

Ces tests ont été conduits de la façon suivante :
- ***Antagonisme à l'hypothermie induite par l'apomorphine***
*(A.J. Puech et al., Psychopharmacology, 1981, 75(1), 84-91*).
On a utilisé des souris mâles CD1 (Charles River, France) d'un poids compris entre 22 et 25 g. Les souris sont placées isolément dans des boites en matière plastique transparente. Les produits à tester ainsi que les produits de comparaison ont été mis en suspension dans une solution aqueuse à 1% de carboxyméthylcellulose. Les produits ont été administrés par voie intrapéritoneale sous le volume de 10 ml/kg (6 souris par dose). Les animaux de contrôle ont reçu seulement le véhicule.
La température rectale a été mesurée à l'aide d'une sonde porteuse d'un couple thermoélectrique (relié à un galvanomètre) enfoncée à une profondeur constante.
Les produits à tester ainsi que le véhicule ont été administrés 30 minutes après la mesure de la température rectale de base et 30 minutes avant l'apomorphine (16 mg/kg sous-cutanée). La température rectale a été mesurée à nouveau 30 minutes après l'administration d'apomorphine.
Les résultats ont été exprimés comme température moyenne de chaque groupe, en évaluant les significations des variations de température entre les groupes de souris traitées et ceux du contrôle à l'aide du test t de Student, et utilisés pour calculer la D.M.A., c'est à dire la dose minimum active.
Les résultats sont présentés dans le TABLEAU I :

**TABLEAU I**

| COMPOSE | Antagonisme à l'hypothermie induite par l'apomorphine 16 mg/kg s.c. D.M.A. mg/kg i.p. |
|---|---|
| Chlorhydrate de N-[(2S)-7-éthoxycarbonylméthoxy-1,2,3,4-tétrahydronapht-2-yl]-(2R)-2-hydroxy-2-(3-chlorophényl)éthanamine (Composé A) | 0,1 (0,3 p.o.) |
| Chlorhydrate de N-[(2R)-7-éthoxycarbonylméthoxy-1,2,3,4-tétrahydronapht-2-yl]-(2R)-2-hydroxy-2-(3-chlorophényl)éthanamine (Composé B) | 0,1 |
| Chlorhydrate de N-[(2S)-7-éthoxycarbonylméthoxy-1,2,3,4-tétrahydronapht-2-yl]-(2S)-2 -hydroxy-2-(3-chlorophényl)éthanamine (Composé C) | 1 |
| Chlorhydrate de N-[(2S)-7-(2-éthoxycarbonyl-propan-2-yloxy)-1,2,3,4-tétrahydronapht-2-yl]-(2R)-2-hydroxy-2-(3-chlorophényl)éthanamine (Composé D) | 0,3 |
| Chlorhydrate de N-[(2R)-7-(2-éthoxycarbonyl-propan-2-yloxy)-1,2,3,4-tétrahydronapht-2-yl]-(2R)-2-hydroxy-2-(3-chlorophényl)éthanamine (Composé E) | 0,3 |
| Clenbutérol | 0,03 |
| Salbutamol | 1 |

- ***Activité motrice.***
Alors que, chez la souris, le clenbutérol et le salbutamol diminuent l'activité motrice qui, à la dose de 0,03 mg/kg de clenbutérol et de 3 mg/kg de salbutamol, n'est plus, respectivement, que de 35 et 62% de celle des témoins, aux doses indiquées ci-dessus les composés (I) enumerés dans le tableau (I) n'ont provoqué presqu'aucune variation de la motricité, le Composé A étant complètement inactif même à la dose de 10 mg/kg i.p.
- ***Antagonisme de l'hypothermie induite par la réserpine***.
Les composés de formule (I) ainsi que les produits de référence ont été administrés 4 heures après la réserpine (2,5 mg/kg, i.p.). La température rectale a été mesurée 90 minutes après l'administration des produits à tester.
Les résultats qui ont été obtenus, à titre d'exemple, avec le Composé A, sont résumés dans le TABLEAU II :

**TABLEAU II**

| COMPOSE | Dose mg/kg i.p. (groupes de 6 à 10 souris par dose) | Température rectale °C (± E.S.M.) 1h30 après administration du composé; 5h30 après injection de réserpine (2,5 mg/kg i.p.) | Dose minimum active mg/kg i.p. |
|---|---|---|---|
| Composé A | 0 | 28,9 ± 0,3 | 0,1 (1 p.o.) |
| | 0,1 | 30,3 ± 0,4* | |
| | 0,3 | 30,7 ± 0,3** | |
| | 1 | 31,4 ± 0,2*** | |
| Clenbutérol | 0 | 32,8 ± 0,3 | 0,03 |
| | 0,03 | 34,0 ± 0,4* | |
| | 0,1 | 35,2 ± 0,4*** | |
| | 0,3 | 34,5 ± 0,3*** | |
| Salbutamol | 0 | 29,9 ± 0,6 | 3 |
| | 1 | 30,5 ± 0,9 | |
| | 3 | 32,6 ± 0,4** | |
| | 10 | 32,0 ± 0,7* | |

| | | | |
|---|---|---|---|
| * = P < 0,05, | | | |
| ** = P < 0,01, | | | |
| *** = P < 0,001 | | | |

- ***Antagonisme de l'hypothermie induite par l'oxotrémorine***.
Les substances à tester ont été administrées 30 minutes avant l'oxotrémorine (0,4 mg/kg, i.p.). La température rectale a été mesurée 30 minutes après l'administration d'oxotrémorine.
L'hypothermie induite par l'administration d'oxotrémorine est antagonisée, au moins partiellement, par les composés de formule (I) ainsi que par les composés de référence.
Avec le Composé A, dont l'activité dans ce test, a été étudiée plus à fond, on a verifié que l'antagonisme progresse régulièrement avec la dose.
- ***Accroissement de la toxicité à la yohimbine.***
L'administration de yohimbine, à la dose de 30 mg/kg sous-cutanée, provoque en moyenne la mort de 1 souris sur 10. Un accroissement de la toxicité est évalué en administrant les produits à tester par voie intra-péritonéale 30 minutes avant la yohimbine et en comptabilisant le pourcentage de mortalité 24 heures après l'injection sous-cutanée de yohimbine. Les composés de formule (I) qui ont été soumis à ce test ont montré une potentialisation nette de la toxicité de la yohimbine à des doses comparables aux doses actives des composés de référence.

En plus, on a maintenant trouvé que les composés de formule (I) exercent une activité anti-stress remarquable.

De nombreuses études récentes ont montrés que le stress peut avoir une influence profonde dans un grand nombre de processus physiologiques. Il est bien connu, par exemple, que l'exposition à des situations créant un stress s'accompagne en général d'une diminution des fonctions immunologiques, des troubles de la motricité intestinale, etc.

L'existence d'un tel lien entre le stress et certains états pathologiques, qu'on indique comme "induits par le stress", augmente l'intérêt thérapeutique des composés de formule (I) qui peuvent donc être utilisés dans le traitement de la dépression ainsi que du stress et donc des états pathologiques induits par le stress.

Pour l'évaluation de l'activité anti-stress on a utilisé des rats mâles CD(BR)SD Charles River (Italie) ayant un poids compris entre 220-250 g et on a provoqué chez eux une situation de stress aigu en les immobilisant complètement, à plat ventre, sur des plaques avec du ruban adhésif. Trois couples d'électrodes ont été implantés de façon permanente sur le côlon (proximal, distal et transverse) des animaux et les fils des électrodes ont été sortis par la région scapulaire et reliés à un électroencéphalographe pour l'enregistrement de l'activité myoélectrique. Comme paramètre de cette activité, on a utilisé le nombre de salves de potentiel (spike-bursts) de longue durée par minute (LSB/min).

Presque immédiatement après l'immobilisation on note une augmentation de la réponse électromyographique du côlon ainsi qu'une augmentation de l'excrétion fécale par rapport aux animaux contrôles non contraints. Ces effets restent plus au moins stables pendant la première heure de stress et diminuent pendant la seconde.

En administrant aux animaux un composé de formule (I) juste avant leur immobilisation, on les protège contre le stress et on remarque ainsi, par rapport aux contrôles contraints, une diminution tant de la réponse électromyographique que de l'excrétion fécale. On obtient cette protection même quand le composé de formule (I) est administré par voie intracranienne à des doses très faibles, ce qui exclut l'existence d'effets périphériques au niveau du côlon. L'effet anti-stress qu'on obtient avec les composés de formule (I) - un effet central au niveau du SNC - peut être rapproché de celui provoqué par les benzodiazépines, en particulier par le diazepam.

Les résultats obtenus avec le Composé A, composé représentatif de formule (I), sont resumés dans les Tableaux III et IV qui suivent.

**TABLEAU III**

| Effets du Composé A et du diazepam sur l'activité myoélectrique du côlon stimulée par le stress chez le rat. | | | | |
|---|---|---|---|---|
| COMPOSE | DOSE mg/kg | Activité myoélectrique du côlon (LSB/min) pendant la première heure de stress | | |
| | | Proximal | Transverse | Distal |
| Contrôles non contraints | - | 1,75 ± 0,06 | 1,10 ± 0,15 | 0,85 ± 0,12 |
| Contrôles contraints | - | 2,41 ± 0,14 ** | 1,75 ± 0,37 * | 1,42 ± 0,25 * |
| Composé A | 0,2 (p.o.) | 1,63 ± 0,11 ° | 1,20 ± 0,15 | 0,67 ± 0,27 ° |
| | 0,5 (p.o.) | 1,07 ± 0,14 °°* | 1,28 ± 0,25 | 0,63 ± 0,12 ° |
| | 2 (p.o.) | 0,76 ± 0,12°°** | 1,16 ± 0,22 | 0,66 ± 0,17 ° |
| | 0,003 (i.c.v.) | 2,07 ± 0,09 | 1,25 ± 0,18 | 0,71 ± 0,15 ° |
| Diazepam | 2 (s.c.) | 1,64 ± 0,12 | 0,99 ± 0,08 | 0,52 ± 0,07 °° |

| | | | | |
|---|---|---|---|---|
| * = P < 0,05 v. contrôles pas contraints | | | | |
| ** = P < 0,01 v. contrôles pas contraints | | | | |
| ° = P < 0,05 v. contrôles contraints | | | | |
| °° = P < 0,01 v. contrôles contraints | | | | |

**TABLEAU IV**

| Effets du Composé A et du diazepam sur l'excrétion fécale induite par le stress chez le rat. | | | |
|---|---|---|---|
| COMPOSE | DOSE mg/kg | Excrétion fécale pendant la première heure de stress | |
| | | poids sec des selles (g ± E.S.) | % inhibition par rapport aux contrôles contraints |
| Contrôles non contraints | - | 0 ± 0 | - |
| Contrôles contraints | - | 1,24 ± 0,05 | - |
| Composé A | 0,2 (p.o.) | 0,80 ± 0,06 ** | 35 |
| | 2 (p.o.) | 0,41 ± 0,07 ** | 67 |
| Diazepam | 2 (s.c.) | 0,51 ± 0,07 ** | 59 |

| | | | |
|---|---|---|---|
| ** = P < 0,01 v. contrôles contraints | | | |

En outre, les composés de formule (I) et leurs sels pharmaceutiquement acceptables possèdent une toxicité très faible, compatible avec l'utilisation de ces produits en tant que médicaments.

Selon la présente invention on préconise donc l'utilisation d'au moins un composé de formule (I) dans laquelle A et R sont tels que définis ci-dessus ou un de ses sels pharmaceutiquement acceptables pour la préparation d'un médicament destiné au traitement ou à la prophylaxie de la dépression et du stress.

Suivant un aspect particulièrement préféré, on préconise l'utilisation des composés de formule (I) ou de leurs sels pharmaceutiquement acceptables pour la préparation d'un médicament destiné au traitement et à la prophylaxie de la dépression.

Selon un autre aspect préféré de l'invention, on préconise l'utilisation des composés de formule (I) ou de ses sels pharmaceutiquement acceptables pour la préparation d'un médicament destiné au traitement ou à la prophylaxie du stress et des états pathologiques induits par le stress, en particulier ceux associés aux troubles de la motricité intestinale.

Par sels pharmaceutiquement acceptables, on entend ici, d'une part les sels d'addition d'acides obtenus à partir de la base libre et d'acides minéraux ou organiques pharmaceutiquement acceptables et d'autre part les sels des composés de formule (I) dans laquelle R est l'hydrogène avec des bases minérales de préférence celles avec des métaux alcalins tels que le sodium ou le potassium, ou avec des bases organiques pharmaceutiquement acceptables.

Parmi les acides utilisables pour salifier la base libre, on peut notamment mentionner les acides chlorhydrique, bromohydrique, acétique, formique, propionique, oxalique, fumarique, maléique, succinique, benzoïque, cinnamique, mandélique, citrique, malique, tartrique, aspartique, glutamique, méthanesulfonique, p-toluènesulfonique, etc.

Le chlorhydrate est de toute façon le sel d'addition acide préféré selon la présente l'invention.

Dans la formule (I) ci-dessus, les deux atomes de carbone asymétriques sont marqués par un astérisque. Tous les composés de formule (I) peuvent donc exister sous forme d'au moins quatre isomères stériques différents [(R,R), (R,S), (S,S) et (S,R)].

Les stéréoisomères optiquement purs, ainsi que les mélanges de deux, trois, ou des quatre isomères, dans une proportion quelconque, peuvent être utilisés pour la préparation de médicaments antidépresseurs selon la présente invention. Un autre centre d'asymétrie pourrait être présent dans le radical A. De même, les stéréoisomères provenant de la présence de ce centre chiral et leurs mélanges peuvent être utilisés selon la présente invention.

Il est de toute façon sous-entendu que, comme il arrive souvent lorsque il s'agit de produits pharmaceutiquement actifs ayant un ou plusieurs centres chiraux, les différents stéréoisomères peuvent avoir des niveaux d'activité différents. S'il le désire, l'homme de métier pourra sur la base des indications données dans le texte de la présente demande et selon son expérience dans le domaine, choisir parmi les differents stéreoisomères ou mélanges d'isomères d'un composé de formule (I), le ou les plus intéressants du point de vue thérapeutique.

Des résultats obtenus ,il ressort que les isomères (I) où l'atome de carbone lié au groupe hydroxy a la configuration absolue (R) sont en général les plus intéressants, et l'utilisation d'au moins un composé de formule (I) où l'atome de carbone lié au groupe hydroxy a la configuration absolue (R), pour la préparation d'un médicament antidépressif et anti-stress représente donc un objet particulièrement préféré de la présente invention.

Un groupe de composés de formule (I) utilisés de préférénce selon la présente invention comprend les composés de formule (I) dans laquelle A est un groupe méthylène ou un groupe isopropylidène. Un groupe plus particulièrement préféré comprend les composés de formule (I) dans laquelle A est un groupe méthylène ou isopropylidène et l'atome de carbone lié au groupe hydroxy a la configuration absolue R.

Les composés encore plus préférés pour l'utilisation selon la présente invention sont les composés de formule (I) dans laquelle A est un groupe méthylène et R est un groupe méthyle ou éthyle.

Pour le traitement ou la prophylaxie de la dépression et du stress, les composés de formule (I) peuvent être administrés par voie orale, sublinguale, transdermique, rectale, sous-cutanée, intramusculaire ou intraveineuse.

La quantité de principe actif à administrer dépend, comme il en est d'habitude, de la nature et de la sévérité des affections à traiter, ainsi que du poids des malades et de la voie d'administration.

La dose par jour chez l'homme varie généralement entre 0,01 et 30 mg par kg de poids du corps, et de préférence entre 0,01 et 10 mg par kg de poids du corps.

Cette dose est normalement subdivisée pendant la journée en 2,3, ou 4 administrations. De préférence, pour le traitement ou la prophylaxie de la dépression ainsi que du stress, les principes actifs (les composés de formule (I) et leurs sels pharmaceutiquement acceptables) sont formulés en unités de dosage contenant de 0,1 à 400 mg, et préférablement de 0,5 à 100 mg de principe actif en combinaison avec un support pharmaceutique.

Des formes unitaires de dosage appropriées pour l'administration orale comprennent les comprimés, les gélules, les poudres, les granules et les solutions ou suspensions orales.

Lorsqu'on prépare une composition solide sous forme de comprimés, on mélange le principe actif principal avec un véhicule pharmaceutique tel que la gélatine, l'amidon, le lactose, le stéarate de magnésium, le talc, la gomme arabique ou analogues. On peut enrober les comprimés de saccharose ou d'autres matières appropriées ou encore on peut les traiter de telle sorte qu'ils aient une activité prolongée ou retardée et qu'ils libèrent d'une façon continue une quantité prédéterminée de principe actif.

On peut obtenir une préparation en gélules en mélangeant le principe actif avec un diluant et en versant le mélange obtenu dans des gélules molles ou dures.

Une préparation sous forme de sirop ou d'élixir ou pour l'administration sous forme de gouttes peut contenir le principe actif conjointement à un édulcorant, acalorique de préférence, du méthylparaben et du propylparaben comme antiseptiques, ainsi qu'un agent donnant du goût et un colorant approprié.

Les poudres ou les granules dispersibles dans l'eau peuvent contenir l'ingrédient actif en mélange avec des agents de dispersion ou des agents mouillants, ou des agents de mise en suspension, comme la polyvinylpyrrolidone, de même qu'avec des édulcorants ou des correcteurs du goût.

Pour une administration rectale, on recourt à des suppositoires qui sont préparés avec des liants fondant à la température rectale, par exemple du beurre de cacao ou des polyéthylèneglycols.

Pour une administration sublinguale on peut préparer des microcomprimés ou microgélules qui se dissolvent rapidement lorsqu'ils sont mis sous la langue. Ces compositions contiennent, en général, le principe actif en mélange avec des agents mouillants et/ou des agents de dispersion et éventuellement des agents qui augmentent l'absorption.

Pour une administration transdermique on préconise l'utilisation des matrices de diffusion polymériques pour la libération continue et préférablement retardée du principe actif, ainsi que l'utilisation du principe actif sous forme de microémulsion avec des excipients appropriés au contact avec la peau.

Pour une administration parentérale, on utilise des suspensions aqueuses, des solutions salines isotoniques ou des solutions stériles et injectables qui contiennent des agents de dispersion et/ou des agents mouillants pharmacologiquement compatibles, par exemple le propylèneglycol ou le butylèneglycol.

Le principe actif principal de formule (I) peut être formulé également sous forme de microcapsules, éventuellement avec un ou plusieurs supports ou additifs.

Le principe actif de formule (I) peut être administré sous forme de base libre ou de sel pharmaceutiquement acceptable tel quel ou sous forme de complexe avec, par exemple, une cyclodextrine ou bien en association ou en co-administration avec d'autres principes actifs.

A titre d'exemple des compositions pharmaceutiques appropriées peuvent être préparés comme décrit ci-dessus.
- On prépare des comprimés à base du chlorhydrate de N-[(2S)-7-éthoxycarbonylméthoxy-1,2,3,4-tétrahydronapht-2-yl]-(2R)-2-hydroxy-2-(3-chlorophényl)éthanamine (Composé A), ayant la composition suivante :

| | |
|---|---|
| Composé A | 5 mg |
| Cellulose microcristalline | 40 mg |
| Amidon de mais séché | 40 mg |
| Lactose | 100 mg |
| Stéarate de magnésium | 5 mg |

en broyant l'ingrédient actif jusqu'à une dimension particulière de 0,4 mm, en le faisant passer à travers un tamis de 0,4 mm d'ouverture de maille, en mélangeant la matière broyée avec les autres constituants et en comprimant pour former les comprimés.
De la même façon, on prépare des comprimés contenant 10 mg d'ingrédient actif.
- On opère substantiellement de la même façon mais en utilisant en tant que principe actif le chlorhydrate de N-[(2S)-7-(2-éthoxycarbonyl-propan-2-yloxy)-1,2,3,4-tétrahydronapht-2-yl]-(2R)-2-hydroxy-2-(3-chlorophényl)éthanamine (Composé D), et on obtient des comprimés ayant la composition suivante :

| | |
|---|---|
| Composé D | 50,0 mg |
| Amidon de maïs séché | 100,0 mg |
| Lactose | 95,0 mg |
| Talc | 4,5 mg |
| Stéarate de magnésium | 0,5 mg |

- 10.000 gélules avec une teneur en substance active de 20 mg sont préparées à partir des constituants suivants: 200 g de Composé A, 990 g de cellulose microcristalline, 10 g de gel de silice amorphe. Les constituants ci-dessus sont bien mélangés et introduits dans des gélules de gélatine dure.
- On prépare une solution aqueuse stérile appropriée pour une utilisation parentérale en ampoules à base du Composé A ayant la composition suivante :

| | |
|---|---|
| Composé A | 5 mg |
| Chlorure de sodium | 1 mg |
| Eau distillée q.s.p. | 2 ml |

## Revendications

1. Utilisation d'au moins un composé de formule (I) 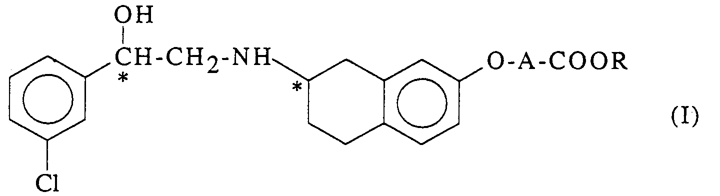 dans laquelle A répresente un groupe (C₁-C₄)alkylène et R répresente un groupe (C₁-C₄)alkyle ou un atome d'hydrogène ou un de ses sels pharmaceutiquement acceptables pour la préparation d'un médicament destiné au traitement ou à la prophylaxie de la dépression et du stress.

2. Utilisation selon la revendication 1, dans laquelle A représente un groupe méthylène ou isopropylidène.

3. Utilisation selon l'une des revendications 1 ou 2, caractérisée en ce que l'atome de carbone lié au groupe hydroxy dans la formule (I) a la configuration absolue R.

4. Utilisation selon l'une quelconque des revendications 1 à 3, caractérisée en ce que R est le groupe méthyle ou le groupe éthyle.

5. Utilisation selon la revendication 4, caractérisée en ce que le composé de formule (I) est choisi entre le N-[(2S) 7-éthoxycarbonylméthoxy-1,2,3,4-tétrahydronapht-2-yl]-(2R)-2-hydroxy-2-(3-chlorophényl)-éthanamine, et ses sels pharmaceutiquement acceptables.

## Claims

1. Use of at least one compound of formula (I) 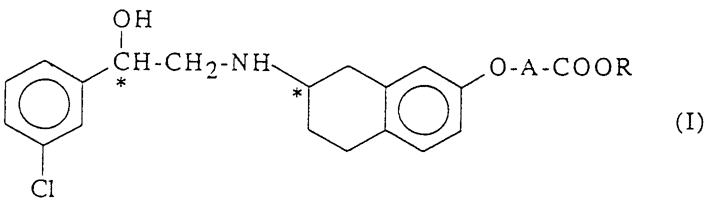 in which A represents a (C₁-C₄)alkylene group and R stands for a hydrogen atom or a (C₁-C₄)alkyl group, or a pharmaceutically acceptable salt thereof for the preparation of a medicament intended for the treatment or prophylaxis of depression and stress.

2. Use according to claim 1, in which A represents a methylene or isopropylidene group.

3. Use according to one of claims 1 or 2, characterized in that the carbon atom linked to the hydroxy group in formula (I) has absolute configuration (R).

4. Use according to any one of claims 1 to 3, characterized in that R is the methyl group or the ethyl group.

5. Use according to claim 4, characterized in that the compound of formula (I) is selected from the N-[(2S) 7-ethoxycarbonylmethoxy-1,2,3,4-tetrahydronaphth-2-yl]-(2R)-2-hydroxy-2-(3-chlorophenyl)ethanamine and the pharmaceutically acceptable salts thereof.

## Patentansprüche

1. Verwendung zumindest einer Verbindung der Formel (I) 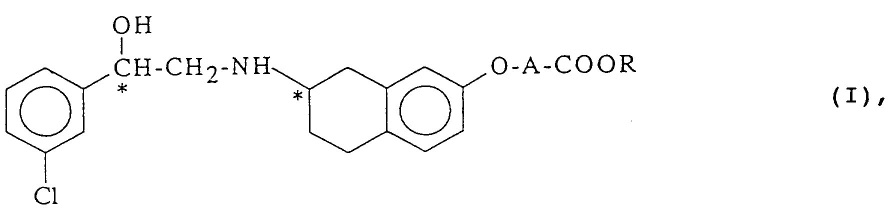 worin A eine C₁-C₄-Alkylen-Gruppe bedeutet, und R eine C₁-C₄-Alkyl -Gruppe oder ein Wasserstoffatom darstellt, oder eines ihrer pharmazeutisch annehmbaren Salze bei der Herstellung eines Medikaments zur Behandlung oder Prophylaxe von Depressionen oder Streß.

2. Verwendung nach Anspruch 1, wobei A eine Methylen- oder Isopropyliden-Gruppe bedeutet.

3. Verwendung nach Anspruch 1 oder 2, dadurch gekennzeichnet, daß das Kohlenstoffatom, das mit der Hydroxy-Gruppe in Formel (I) verbunden ist, absolute (R)-Konfiguration aufweist.

4. Verwendung nach einem der Ansprüche 1 bis 3, dadurch gekennzeichnet, daß R eine Methyl- oder Ethyl-Gruppe ist.

5. Verwendung nach Anspruch 4, dadurch gekennzeichnet, daß die Verbindung der Formel (I) ausgewählt ist aus N-[(2S)-7-Ethoxycarbonylmethoxy-1,2,3,4-tetrahydronaphth-2-yl]-(2R)-2-hydroxy-2-(3-chlorphenyl)-ethanamin und seinen pharmazeutisch annehmbaren Salzen.
